# EUROPEAN PATENT APPLICATION

(11) **EP 4 344 668 A1**
(43) Date of publication of application: **03.04.2024**
(21) Application number: 23199423.7
(22) Date of filing: 25.09.2023
(51) Int. Cl.: A61B 34/37, A61B 34/35, A61B 90/00

(54) **REMOTE SURGERY SUPPORT SYSTEM**

(30) Priority: 28.09.2022 JP 2022155158
(71) Applicant: Medicaroid Corporation, Kobe-shi, Hyogo 650-0047 (JP)
(72) Inventor: KITATSUJI, Hiroaki, Hyogo, 650-0047 (JP); OHASHI, Masanao, Hyogo, 650-0047 (JP)
(74) Representative: Müller Hoffmann & Partner

(57) **Abstract**

A remote surgery support system according to one or more embodiments may include: a doctor-side operating device in a first facility to operate manipulator arms in the first facility; a mentor-side operating device in a second facility to operate the manipulator arms via an external network; and one or more controllers. The doctor-side operating device includes a first display to display an endoscopic image and a first voice communication device. The mentor-side operating device includes: a second display to display the endoscopic image; a second voice communication device for performing voice communication with the first voice communication device; a touch panel display to display the endoscopic image and receive an instruction input for generating an annotation image; and a third voice communication device provided near the touch panel display device for performing voice communication with the first voice communication device. The one or more controllers is configured to display on the first display the annotation image based on the instruction input received by the touch panel display with the annotation image superimposed with the endoscopic image.

## Description

### BACKGROUND

The disclosure may relate to a remote surgery support system and may particularly relate to a remote surgery support system that allows voice communication between a doctor and a mentor.

In a related art, there has been known a remote surgery support system that allows voice communication between a surgeon and a mentor surgeon (e.g., see Patent Document 1).

The medical robot system (the remote surgery support system) disclosed in Patent Document 1 comprises a surgeon master control station to be operated by the surgeon who operates surgery, a slave cart with a plurality of slave robot mechanisms, and a mentor master control station to be operated by a mentor. In the medical robot system, the surgeon and the mentor use headsets to perform voice communication between the surgeon master control station and the mentor master control station. The mentor also performs telestration (tele-illustration) using a touch screen connected to the mentor master control station to generate illustrations to be superimposed on an anatomy image.

Patent Document 1: Japanese Patent Application Publication No. 2007-181670

Non-Patent Document 1: Remote Surgery Guidelines, Japan Surgical Society, June 22, 2022 edition

### SUMMARY

However, in Patent Document 1, it is not mentioned to place the mentor master control station in a separate facility from a facility where the surgeon master control station and the slave cart are located, and use the system via a network. In addition, upon performing the "remote surgery support" described in Remote Surgery Guidelines (Non-Patent Document 1) via a network in a state where the mentor master control station is placed in a separate facility from a facility where the surgeon master control station and the slave cart are placed, it is necessary to place a clinical engineer on the mentor side. However, in Patent Document 1, since the mentor master control station is configured to perform voice communicate using the headset by the mentor, the mentor can perform telestration (annotation) while talking with the surgeon during surgery, but the clinical engineer cannot talk with the surgeon side.

An object of an embodiment of the disclosure is to provide a remote surgery support system that allows a mentor to annotate while interacting with a surgeon during surgery and allow the clinical engineer as a remote surgery staff on the mentor side to interact with the surgeon side.

A first aspect of this disclosure may be a remote surgery support system that may include: a first manipulator arm placed in a first facility and holding a first surgical instrument; a second manipulator arm placed in the first facility and holding a second surgical instrument; a third manipulator arm placed in the first facility and holding an endoscope; a doctor-side operating device placed in the first facility and configured to be operated by a doctor to operate the first to third manipulator arms; a mentor-side operating device placed in a second facility different from the first facility and configured to be operated by a mentor to operate the first to third manipulator arms via an external network; and one or more controllers. The doctor-side operating device includes a first display configured to display an endoscopic image acquired by the endoscope, and a first voice communication device. The mentor-side operating device includes: a second display configured to display the endoscopic image transmitted through the external network; a second voice communication device provided at a position that allows the mentor to perform voice communication with the first voice communication device while viewing the endoscopic image displayed on the second display; a touch panel display configured to display the endoscopic image transmitted through the external network and receive an instruction input for generating an annotation image; and a third voice communication device provided at a position closer to the touch panel display than the second display and configured to perform voice communication with the first voice communication device. The one or more controllers is configured to display on the first display the annotation image based on the instruction input received by the touch panel display with the annotation image being superimposed with the endoscopic image.

According to the first aspect described above, by accepting the instruction input through the touch panel display and the voice communication between the third voice communication device and the first voice communication device, it is possible to perform the annotation while the mentor interacts with the doctor during the operation. In addition, through the voice communication between the third voice communication device and the first voice communication device, the clinical engineer who is assigned as a remote surgical staff on the mentor side can communicate with the doctor. As a result, it is possible for the mentor to perform the annotation while interacting with the doctor during surgery, and for the clinical engineer who is assigned as a remote surgical staff on the mentor side to communicate with the doctor side.

A second aspect of this disclosure may be a mentor-side operating device. The mentor-side operating device is placed in a second facility different from a first facility in which a surgical system is placed, the surgical system of the first facility includes a doctor-side operating device that includes: a first manipulator arm holding a first surgical instrument; a second manipulator arm holding a second surgical instrument; a third manipulator arm holding an endoscope; a first display configured to display an endoscopic image acquired by the endoscope; and a first voice communication device. The mentor-side operating device is configured to operate via an external network from the second facility the first to third manipulator arms of the surgical system in the first facility. The mentor-side operating device includes: a second display configured to display the endoscopic image transmitted through the external network; a second voice communication device provided at a position that allows a mentor to perform voice communication with the first voice communication device while viewing the endoscopic image displayed on the second display; a touch panel display configured to display the endoscopic image transmitted through the external network and to receive an instruction input for generating an annotation image; and a third voice communication device provided at a position closer to the touch panel display than to the second display for performing voice communication with the first voice communication device. The mentor-side operating device is configured to transmit via the external network to the doctor-side operating device the instruction input received by the touch panel display, for displaying on the first display the annotation image superimposed with the endoscopic image.

According to the second aspect described above, the reception of the instruction input through the touch panel display along with the voice communication between the third voice communication device and the first voice communication device, it is possible for the mentor to annotate while interacting with the doctor during the operation. In addition, through the voice communication between the third voice communication device and the first voice communication device, the clinical engineer who is assigned as a remote surgical staff on the mentor side can communicate with the doctor side. As a result, it is possible to provide the mentor-side operating device that allows the mentor to annotate while interacting with the doctor during surgery and allows the clinical engineer who is assigned as a remote surgical staff on the mentor side to communicate with the doctor side.

According to at least one of the aspects described above, it is possible for the mentor to annotate while interacting with the doctor during surgery, and for the clinical engineer assigned as a remote surgical staff on the mentor side to interact with the doctor side.

### BRIEF DESCRIPTION OF DRAWINGS

FIG. 1 is a diagram illustrating a view of a configuration of a remote surgery support system according to an embodiment;
FIG. 2 is a diagram illustrating a view of a configuration of a surgical robot according to an embodiment;
3 is a diagram illustrating a view of a configuration of a doctor-side operating device (a mentor-side operating device) according to an embodiment;
FIG. 4 is a block diagram illustrating a view of a configuration of the remote surgery support system according to an embodiment;
FIG. 5 is a diagram illustrating a view of a scope type display of the remote surgery support system according to an embodiment;
FIG. 6 is a block diagram illustrating a view of a configuration of a controller of an information sharing device according to an embodiment;
FIG. 7 is a diagram illustrating a view of an endoscopic image on which an annotation image is superimposed according to an embodiment;
FIG. 8 is a diagram for explaining a switching request of an operation authority to operate the surgical robot according to an embodiment;
FIG. 9 is a diagram for explaining a notification of the switching request of the operation authority according to an embodiment;
FIG. 10 is a diagram for explaining a response to the switching request of the operation authority according to an embodiment;
FIG. 11 is a diagram for explaining a display of the operation authority according to an embodiment;
FIG. 12 is a flowchart for explaining a control process for switching the operation authority according to an embodiment; and
FIG. 13 is a block diagram illustrating a view of a configuration of a remote surgery support system according to a modification.

### DETAILED DESCRIPTION

Descriptions are provided hereinbelow for embodiments based on the drawings. In the respective drawings referenced herein, the same constituents are designated by the same reference numerals and duplicate explanation concerning the same constituents is omitted. All of the drawings are provided to illustrate the respective examples only.

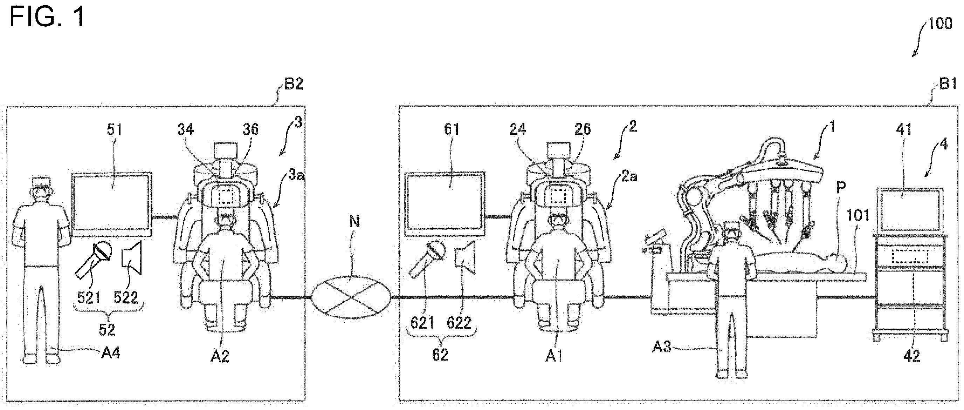

A configuration of a remote surgery support system 100 (a remote surgical system, a telesurgery system) according to an embodiment is described with reference to FIGS. 1 to 12.

As illustrated in FIG. 1, the remote surgery support system 100 includes: a surgical robot 1 placed in the vicinity of an operation table 101 on which a patient P is to be placed; a doctor-side operating device 2 through which a doctor A1 (surgeon A1) operates the surgical robot 1; and a mentor-side operating device 3 through which a mentor A2 (such as a guidance doctor, an experienced doctor, a supervising doctor, an instructing doctor, a preceptor, a proctor, or the like) operates the surgical robot 1 via an external network N. The surgical robot 1 and the doctor-side operating device 2 are located at a first facility B1. The mentor-side operating device 3 is placed in a second facility B2 different from the first facility B1. In the first facility B1, an assistant(s) A3 as a surgical staff(s) is assigned near the surgical robot 1. Further, in the first facility B1, an information sharing device 4 for sharing information with the assistants A3 is placed. In the second facility B2, a clinical engineer A4 is assigned near the mentor-side operating device 3 as a remote surgical staff.

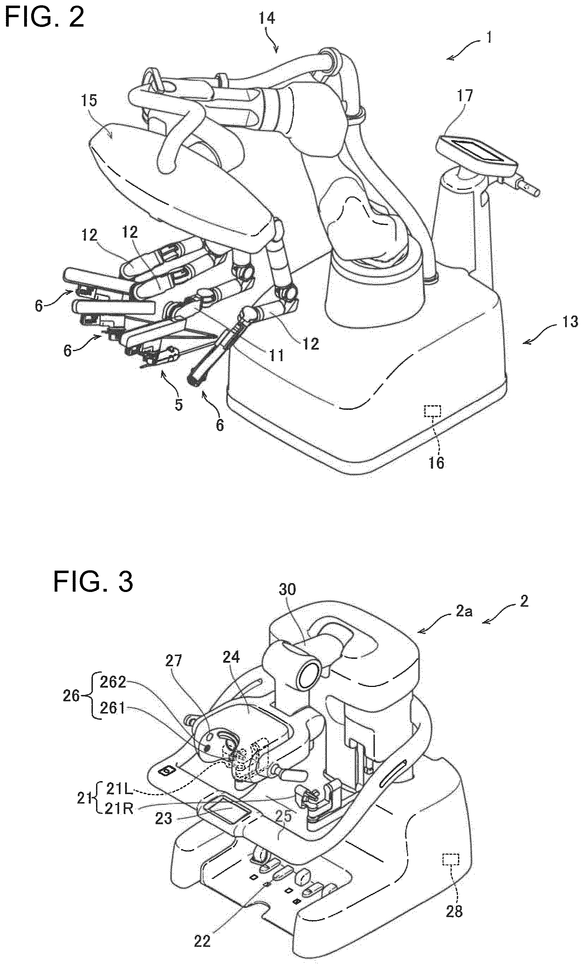

As illustrated in FIG. 2, the surgical robot 1 includes one manipulator arm 11 that holds an endoscope 5 and a plurality (three, in this example) of manipulator arms 12 that respectively hold surgical instruments 6. The endoscope 5 captures an image of a surgical site inside a patient P. An end effector such as forceps or the like is attached to a distal end of each of or at least one of the surgical instruments 6. The doctor-side operating device 2 and the mentor-side operating device 3 are provided for operating the manipulator arms 11 and 12. Note that the manipulator arm 11 and the manipulator arm 12 are examples of a "first manipulator arm" and a "second manipulator arm", respectively.

The surgical robot 1 is placed in a surgery room (an operating room) in the first facility B1. The surgical robot 1 includes a medical cart 13, a positioner 14 and an arm base 15. The surgical robot 1 is configured to be movable by the medical cart 13. The medical cart 13 is provided with a controller 16 (or a control device) that controls operation of the surgical robot 1. The controller 16 controls the operation of the surgical robot 1 based on a command input to the doctor-side operating device 2 or the mentor-side operating device 3. The controller 16 includes a processor or a circuitry such as a CPU or the like that executes programs, and a storage such as a memory that stores the programs. Note that the controller 16 is an example of "one or more controllers" or "one or more control devices".

Further, the medical cart 13 is provided with an input device 17. The input device 17 is configured to accept operations to move or change postures of the manipulator arm 11, the manipulator arms 12, the positioner 14, the arm base 15, mainly in order to prepare for surgery before the surgery.

The positioner 14 is configured as a seven-axis articulated robot. The positioner 14 is disposed on the medical cart 13. The positioner 14 is configured to move the arm base 15. Specifically, the positioner 14 is configured to move the position of the arm base 15 three-dimensionally.

The arm base 15 is attached to a distal end of the positioner 14. The arm base 15 is a relatively long rod shape (elongate shape). The manipulator arm 11 and the manipulator arms 12 are attached to the arm base 15 at their base portions (proximal end portions). The arm base 15, manipulator arm 11, and manipulator arms 12 are used with being covered with a sterile drape.

As illustrated in FIG. 1, the doctor-side operating device 2 is located, for example, in or outside the surgery room (the operating room) in the first facility B1. The doctor-side operating device 2 includes a doctor-side operating device main body 2a. As illustrated in FIG. 3, the doctor-side operating device main body 2a includes operating handles 21, foot pedals 22, a touch panel 23, a scope type display 24, an armrest 25, a voice communication device 26, a sensor 27, a controller 28 (a control device 28), and a support arm 30. The operating handle 21 is an example of a "first operating handle". The touch panel 23 is an example of a "switching device." The scope type display 24 is an example of a "first display" or a "first display device." The voice communication device 26 is an example of a "first voice communication device."

The operating handles 21 are control handles provided for an operator (doctor A1) to input commands. The operating handles 21 are provided for the operator (doctor A1) to operate the manipulator arms 11 and 12 (the endoscope 5 and the surgical instruments 6). Each of the operating handles 21 also receives an operation amount (a movement amount) by which the operating handle 21 is operated (moved) to operate the manipulator arms 11 and 12 (the endoscope 5 and the surgical instruments 6). The operating handles 21 comprise a pair of operating handles 21 which include a left-hand operating handle 21L to be operated by the left hand of the operator (doctor A1) and a right-hand operating handle 21R to be operated by the right hand of the operator (doctor A1). The surgical instrument 6 that is held by one manipulator arm 12 is operated by the operating handle 21L, and the surgical instrument 6 that is held by another manipulator arm 12 is operated by the operating handle 21R.

Further, a movement amount of the endoscope 5 or the surgical instrument 4 is scaled (changed) with respect to the operation amount received by the operating handle 21. For example, when the magnification of the movement amount (the scaling ratio) is set to 1/2, the endoscope 5 or the surgical instrument 6 move 1/2 of the movement distance of the operating handle 21. This allows for precise fine surgery.

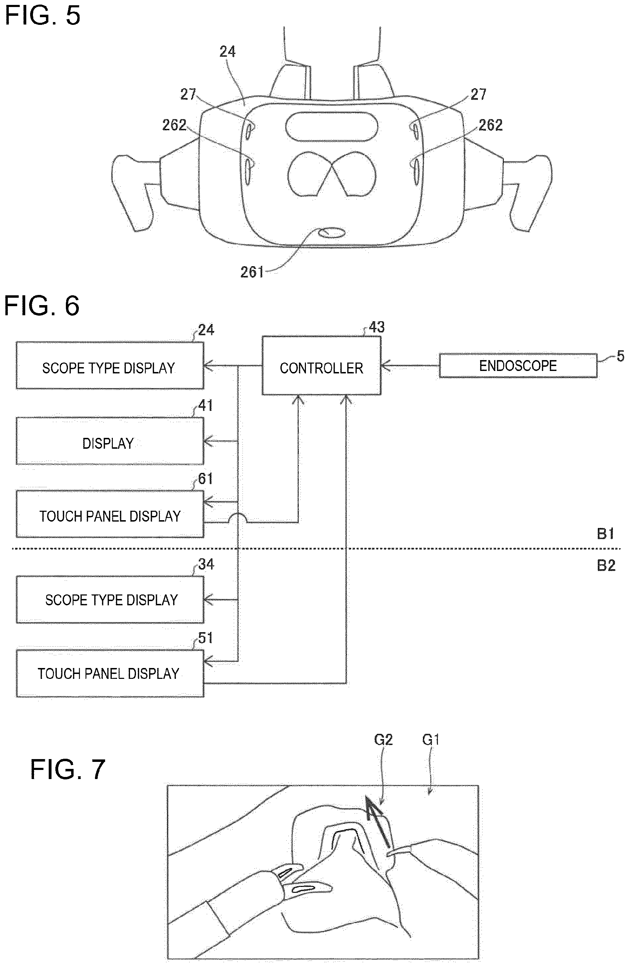

The foot pedals 22 include a plurality of pedals to perform functions related to the endoscope 5 and the surgical instruments 6. The plural pedals include a coagulation pedal, a cutting pedal, a camera pedal, and a clutch pedal. The plurality pedals are operated by a foot (feet) of the operator (doctor A1). When the camera pedal is operated, the operating handles 21 are allowed to move the endoscope 5 held by the manipulator arm 11. At this time, the endoscope 5 is moved by operating both the operating handle 21L and the operating handle 21R. The touch panel 23 is configured to accept setting operations regarding the doctor-side operating device 2. The touch panel 23 is provided to the armrest 25. The scope type display 24 is configured to display an endoscopic image G1 (see FIG. 7) acquired by the endoscope 5. The scope type display 24 is an immersive display that allows only the operator (doctor A1) to see the displayed image. The operator (doctor A1) operates the operating handles 21 and the foot pedals 22 while viewing a surgical site (or an affected area) displayed on the scope type display 24. The support arm 30 supports the scope type display 24 so that the height of the scope type display 24 can be adjusted to the height of the face of the operator such as a doctor A1.

The armrest 25 is formed in a shape of a bar, and is configured so that the operator (doctor A1) can put his or her arms thereon when operating the operating handles 21. The voice communication device 26 is provided at a position where the operator (doctor A1) can perform voice communication with a voice communication device 36 (described later) of the mentor-side operating device 3 while looking into the scope type display 24. Specifically, the voice communication device 26 is provided near the head of the operator (doctor A1) when the operator (doctor A1) looks into the scope type display 24. The voice communication device 26 is provided integrally with the scope type display 24 and thus the voice communication device 26 and the scope type display 24 can be integrally adjusted in height by the support arm 30.

The sensor 27 is configured to detect whether or not the scope type display 24 is in a state of use in which the scope type display 24 is looked into. The sensor 27 is provided at a position where the sensor 27 detects the head of the operator (doctor A1) when the operator positions the head to look into the scope type display 24. The sensor 27 is provided integrally with the scope type display 24. The controller 28 controls the operation of the doctor-side operating device 2. The controller 28 includes a processor or a circuitry such as a CPU or the like that executes programs, and a storage such as a memory that stores the programs.

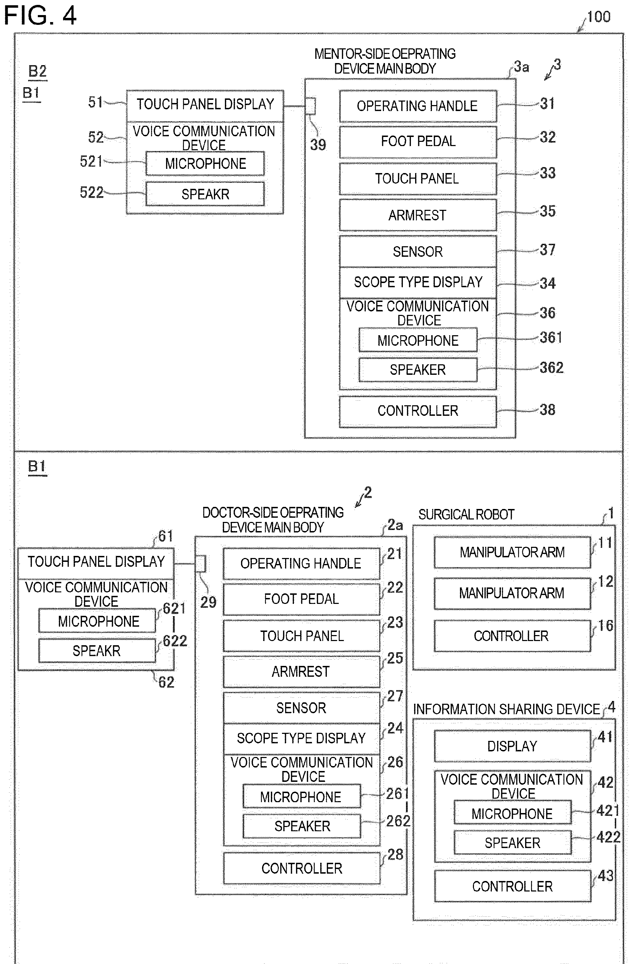

Although the doctor-side operating device 2 has been described above, the mentor-side operating device 3 has the structure same as or similar to the doctor-side operating device 2. That is, as illustrated in FIG. 4, the mentor-side operating device 3 includes a mentor-side operating device main body 3a. The mentor-side operating device main body 3a includes operating handles 31, foot pedals 32, a touch panel 33, a scope type display 34, an armrest 35, the voice communication device 36, a sensor 37, a controller 38, and a support arm. The scope type display 34 is configured to display the endoscopic image G1 (see FIG. 7) acquired by the endoscope 5 and transmitted via the external network N (see FIG. 1). Although the doctor-side operating device 2 and the mentor-side operating device 3 are basically the same specifications, the normal mode and the remote mode are switched via the touch panel 23 (33). The operating handle 31 is an example of a "second operating handle". The touch panel 33 is an example of a "switching device." The voice communication device 36 is an example of a "second voice communication device."

In an embodiment, as illustrated in FIGS. 1 and 4, the mentor-side operating device 3 includes: a touch panel display 51 configured to display the endoscopic image G1 transmitted through the external network N (see FIG. 7) and to receive an instruction input for generating the annotation image G2 (see FIG. 7); and a voice communication device 52 disposed in the vicinity of the touch panel display 51 closer to the touch panel display 51 than the scope type display 34 so as to perform voice communication with the voice communication device 26. Further, in order to display the annotation image G2 on the scope type display 24, the controller 38 of the mentor-side operating device 3 transmits the instruction input received by the touch panel display 51, through the external network N to the doctor-side operating device 2. Further, a controller 43 (to be described later) of the information sharing device 4 displays the annotation image G2 based on the instruction input received by the touch panel display 51 with superimposing the annotation image G2 on the endoscopic image G1 on the scope type display 24, as illustrated in FIG. 7. Thus, with the voice communication between the voice communication device 52 and the voice communication device 26 and the acceptance of the instruction input through the touch panel display 51, it is possible for the mentor A2 to perform annotation while interacting with the doctor A1 during the operation. In addition, through the voice communication between the voice communication device 52 and the voice communication device 26, the clinical engineer A4 who is assigned as a remote surgical staff on the mentor A2 side can communicate with the doctor A1 side. As a result, it is possible for the mentor A2 to annotate while interacting with the doctor A1 during surgery and for the clinical engineer A4 who is assigned as a remote surgical staff on the mentor A2 side to communicate with the doctor A1 side. Further, since the mentor A2 can annotate without looking into the scope type display 34, it is possible to reduce the burden on the body of the mentor A2 as compared with a case where the mentor A2 annotates while looking into the scope type display 34. Note that the mentor A2 can interact with the doctor A1 through voice communication between the voice communication device 36 and the voice communication device 26. The mentor A2 can also interact with the clinical engineer A4 through voice communication between the voice communication device 36 and the voice communication device 52. This allows the mentor A2 to communicate with the doctor A1 and to communicate with the clinical engineer A4 located on the mentor A2 side. Note that the controller 43 is an example of "one or more controllers."

The touch panel display 51 and the voice communication device 52, as an annotation unit or an annotation device independent from the mentor-side operating device main body 3a, are arranged in the vicinity of the mentor-side operating device main body 3a. The touch panel display 51 is, for example, a flat panel display having a touch panel function. The mentor A2 causes the touch panel display 51 to receive the instruction input using a finger or a pen for a touch panel to generate an annotation image G2 having a shape corresponding to the instruction input. The annotation image G2 may be, for example, a line(s), a circle(s), an arrow(s), or the like.

Also, in an embodiment, as illustrated in FIGS. 4 and 5, each of the voice communication device 26, the voice communication device 36, and the voice communication device 52 includes a microphone (261, 361, 521) and a speaker (262, 362, 522). Thus, it is possible to easily perform voice communication (input and output of audio) by the voice communication device 26, the voice communication device 36, and the voice communication device 52. The microphone 261 (361, 521) accepts audio input. The speaker 262 (362, 522) outputs audio. As illustrated in FIG. 5, the speaker 262 (362) is provided at a position close to the operator's ear when the operator (doctor A1 or mentor A2) positions the operator's head to look into the scope type display 24 (34). The microphone 261 (361) is provided at a position close to the operator's mouth when the operator (doctor A1 or mentor A2) looks into the scope type display 24 (34). The microphone 261 (361) and the speaker 262 (362) are provided integrally with the scope type display 24 (34).

Further, in an embodiment, the voice communication device 52 is provided integrally with the touch panel display 51. Thus, it is possible to save the placement space of the voice communication device 52 and the touch panel display 51 as compared with a case where the voice communication device 52 is provided separately from the touch panel display 51. The voice communication device 52 includes, for example, the microphone 521 and the speaker 522 in the touch panel display 51. Note that, in FIG. 1, the touch panel display 51 and the voice communication device 52 are separately illustrated.

Further, in an embodiment, the mentor-side operating device 3 (the mentor-side operating device main body 3a) includes an interface 39 (see FIG. 4) to which an external device is to be connected. The touch panel display 51 is connected to the mentor-side operating device 3 (the mentor-side operating device main body 3a) via the interface 39. Thus, it is possible to connect the voice communication device 52 and the touch panel display 51 to the external network N via the mentor-side operating device 3 (the mentor-side operating device main body 3a). As a result, this can simplify the communication configuration. The interface 39 is a terminal capable of inputting and outputting image data and audio data. The touch panel display 51 is connected to the interface 39 through a cable capable of inputting and outputting image data and voice data. The touch panel display 51 and the voice communication device 52 input and output image data and voice data through the interface 39.

Further, the doctor-side operating device 2 includes an annotation unit or an annotation device in the same manner as in the mentor-side operating device 3. That is, the doctor-side operating device 2 includes: a touch panel display 61 configured to display the endoscopic image G1 (see FIG. 7) and configured to accept the instruction input for generating the annotation image G2 (see FIG. 7); and a voice communication device 62 arranged closer to the touch panel display 61 than the scope type display 24 so as to perform voice communication with the voice communication devices 36 and 52. With the touch panel display 61, it is possible to perform the annotation in both directions between the doctor A1 and the mentor A2 during the operation. Further, with the voice communication device 62, the doctor A1 can also annotate while interacting with the mentor A2 during the operation.

The touch panel display 61 and the voice communication device 62, as an annotation unit or an annotation device independent from the doctor-side operating device main body 2a, is disposed in the vicinity of the doctor-side operating device main body 2a. The touch panel display 61 is, for example, a flat panel display having a touch panel function. The doctor A1 causes the touch panel display 61 to receive the instruction input using a finger or a pen for a touch panel to generate an annotation image G2 having a shape corresponding to the instruction input. The annotation image G2 may be, for example, a line(s), a circle(s), an arrow(s), or the like.

The voice communication device 62 also includes a microphone 621 and a speaker 622. The microphone 621 accepts voice input. The speaker 622 outputs sound. The voice communication device 62 is integrally provided with the touch panel display 61. The voice communication device 62 includes, for example, the microphone 621 and the speaker 622 in the touch panel display 61. Note that, in FIG. 1, the touch panel display 61 and the voice communication device 62 are separately illustrated.

The doctor-side operating device 2 (the doctor-side operating device main body 2a) includes an interface 29 (see FIG. 4) for connecting an external device. The touch panel display 61 is connected to the doctor-side operating device 2 (the doctor-side operating device main body 2a) via the interface 29. The interface 29 is a terminal capable of inputting and outputting image data and audio data. The touch panel display 61 is connected to the interface 29 through a cable capable of inputting and outputting image data and voice data. The touch panel display 61 and the voice communication device 62 input and output image data and voice data through the interface 29.

As illustrated in FIGS. 1 and 4, the information sharing device 4 is a movable monitor cart. With reference to FIGS. 1 and 2, the information sharing device 4 includes a display 41 (a display device) configured to display the endoscopic image G1 (see FIG. 7) acquired by the endoscope 5 (see FIG. 2), and the voice communication device 42 for voice communication with the voice communication device 26 of the doctor-side operating device 2. The information sharing device 4 is located closer to the surgical robot 1 than the doctor-side operating device 2. Therefore, the assistant A3 placed as a surgical staff near the surgical robot 1 can check the endoscopic image G1 through the monitor cart 4 and interact with the doctor A1. The voice communication device 42 is also configured to perform voice communication with the voice communication device 36 and the voice communication device 52. This allows the mentor A2 and the clinical engineer A4 to hear the dialogue between the doctor A1 and the assistant A3 as a surgical staff assigned near the surgical robot 1. The assistant A3 can also interact with the mentor A2 and the clinical engineer A4. The voice communication device 42 also includes a microphone 421 and a speaker 422. The microphone 421 accepts voice input. The speaker 422 outputs sound. The display 41 is, for example, a flat panel display. Note that the display 41 is an example of a "second display" or a "second display device." The voice communication device 42 is an example of a "fourth voice communication device."

Further, the controller 43 is disposed in the information sharing device 4. The controller 43 performs image processing for the endoscopic image G1 and the annotation image G2. The controller 43 includes a processor or a circuitry such as a CPU that executes programs, and a storage such as a memory that stores the programs. Further, in an embodiment, the controller 43 is disposed in the first facility B1. As illustrated in FIGS. 6 and 7, The controller 43 is configured to display the annotation image G2 generated based on the instruction input through the touch panel display 51 and transmitted via the external network N and/or the annotation image G2 generated based on the instruction input through the touch panel display 61, with the annotation image(s) G2 being superimposed on the endoscopic image G1 on the scope type display 24. Thus, it is not necessary to provide, in addition to the controller 43, a controller configured to perform image processing to superimpose the annotation image G2 on the endoscopic image G1 in the second facility B2. Therefore, it is possible to simplify the configuration of the second facility B2.

Note that the controller 43 displays the same image on the scope type display 24, the scope type display 34, the display 41, the touch panel display 51, and the touch panel display 61. That is, the scope type display 24, the scope type display 34, the display 41, the touch panel display 51, and the touch panel display 61 displays the same endoscopic image G1 or the same annotation image G2 superimposed on the same endoscopic image G1.

Note that the voice communication device 26, the voice communication device 36, the voice communication device 42, the voice communication device 53, and the voice communication device 62 are configured to perform voice communication with each other.

Further, in an embodiment, as illustrated in FIGS. 3 to 5, the mentor-side operating device 3 includes the sensor 37 configured to detect whether or not the mentor-side operating device 3 is in the state of use in which the scope type display 34 is looked into, as described above. The controller 16 is configured, when the sensor 37 does not detect the state of use, to prohibit the manipulator arms 11 and 12 from being operated by the operating handles 31, and when the sensor 37 detects the state of use, to permit the manipulator arms 11 and 12 to be operated by the operating handles 31. As a result, when it is detected that the mentor-side operating device 3 is not in the state of use, the manipulator arms 11 and 12 are prevented from being operated (moved) even if the mentor A2 unintentionally touches the operating handles 31. The controller 16 is configured to, in a state where the sensor 37 does not detect the state of use, prohibit the operations of the manipulator arms 11 and 12 by not accepting input from the operating handles 31. Further, the controller 16 is configured to, in a state where the sensor 37 detects the state of use, accept predetermined inputs from the operating handles 31 so as to allow the operating handles 31 to operate the manipulator arms 11 and 12.

Note that the same applies to the doctor-side operating device 2. That is, the controller 16 is configured, when the sensor 27 does not detect the state of use, to prohibit the manipulator arms 11 and 12 from being operated by the operating handles 21, and configured, when the sensor 27 detects the state of use, to permit the manipulator arms 11 and 12 to be operated by the operating handles 21.

Further in an embodiment, each of the doctor-side operating device 2 and the mentor-side operating device 3 is provided with a switching device configured to switch an operation authority (permission) for operating the surgical robot 1 between the doctor-side operating device 2 and the mentor-side operating device 3. With this configuration, the operation authority can be easily switched by using the switching devices at any time when the operation authority is desired to be switched. Further in an embodiment, the switching devices are provided in the armrests 25 and 35. Thus, the switching devices can be located in positions where the doctor A1 and the mentor A2 are easy to operate (easy to place the hands). As a result, the operation to switch the operation authority can be easily performed. The switching device is the touch panel 23 (33) disposed on the armrest 25 (35).

Further, in an embodiment, the controller 16 is configured, when the doctor-side operating device 2 has the operation authority, to permit the instruction input for generating the annotation image G2 through the touch panel display 51. Thus, when the doctor-side operating device 2 operates the surgical robot 1, the annotation image G2 generated with the touch panel display 51 is displayed on the scope type display 24 (and other displays).

Further, in an embodiment, the controller 16 is configured, when the mentor-side operating device 3 has the operation authority, to prohibit acceptance of the instruction input through the touch panel display 51. Thus, when the mentor-side operating device 3 operates the surgical robot 1, it is possible to prohibit acceptance of the instruction input for generating the annotation image G2 through the touch panel display 51. As a result, when the mentor A2 operates the surgical robot 1, the mentor A2 can concentrate on the operation of the surgical robot 1.

The same applies to the annotation image G2 by the touch panel display 61. That is, the controller 16 is configured to, when the mentor-side operating device 3 has the operation authority, allow acceptance, through the touch panel display 61, of an instruction input for generating the annotation image G2. Further, the controller 16 is configured to, when the doctor-side operating device 2 has the operation authority, prohibit acceptance, through the touch panel display 61, of an instruction input for generating the annotation image G2.

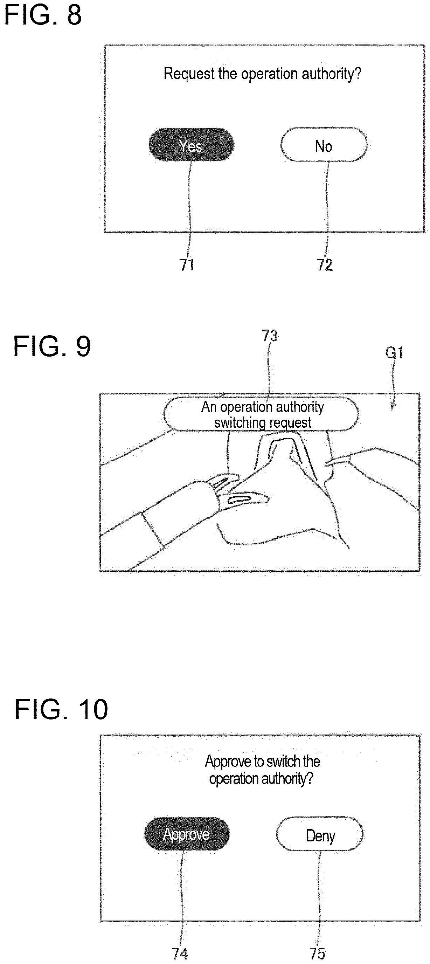

As illustrated in FIGS. 8 to 10, the doctor-side operating device 2 and the mentor-side operating device 3 send a request for switching the operation authority from one of the doctor-side operating device 2 and the mentor-side operating device 3 that does not have the operation authority to the other of the doctor-side operating device 2 and the mentor-side operating device 3 that has the operation authority, and when the other having the operation authority approves the request for switching the operation authority, the operation authority is switched from the one having the operation authority to the other having no operation authority. Thus, since the operation authority is switched after obtaining the approval from one that has the operation authority, it is possible to suppress the occurrence of unintended switching of the operation authority.

As illustrated in FIG. 8, in a case of switching the operation authority between the doctor-side operating device 2 and the mentor-side operating device 3, one of the doctor-side operating device 2 and the mentor-side operating device 3 that does not have the operation authority displays on the touch panel (23, 33) buttons 71 and 72 for selecting whether or not to send the switching request for the operation authority. One of the doctor-side operating device 2 and the mentor-side operating device 3 that does not have the operation authority transmits, via the external network, the switching request for the operation authority to the other of the doctor-side operating device 2 and the mentor-side operating device 3 that has the operation authority, when the button 71 for transmitting the switching request for the operation authority is selected on the touch panel (23, 33).

As illustrated in Fig. 9, one of the doctor-side operating device 2 and the mentor-side operating device 3 that has the operation authority notifies, when receiving the switching request for the operation authority, the notification that indicates the switching request for the operation authority has been received. For example, one of the doctor-side operating device 2 and the mentor-side operating device 3 that has the operation authority displays, on the scope type display (24, 34), an indication 73 notifying that a request for switching the operation authority has been received, with being superimposed on the endoscopic image G1. Note that the process of superimposing the indication 73 on the endoscopic image G1 is performed by the controller 43.

Further, as illustrated in FIG. 10, one of the doctor-side operating device 2 and the mentor-side operating device 3 that has the operation authority displays on the touch panel (23, 33) thereof buttons 74 and 75 for selecting whether or not to approve the switching request for the operating authority. When the button 74 for approving the request for switching the operation authority is selected on the touch panel (23, 33), one of the doctor-side operating device 2 and the mentor-side operating device 3 that has the operation authority transfers the operation authority to the other of the doctor-side operating device 2 and the mentor-side operating device 3 that does not have the operation authority.

To the contrary, when the button 75 for denying the switching request for the operation authority is selected on the touch panel (23, 33), the one of the doctor-side operating device 2 and the mentor-side operating device 3 that has the operation authority transmits the notification of denial of the operation authority switching request to the other of the doctor-side operating device 2 and the mentor-side operating device 3 that does not have the operation authority. In this case, the other of the doctor-side operating device 2 and the mentor-side operating device 3 that does not have the operation authority notifies that the notification of denial of the operation authority switching has been received. For example, the other of the doctor-side operating device 2 and the mentor-side operating device 3 that does not have the operation authority displays, on the scope type display (24, 34) thereof, an indication indicating that the notification of denial of the operation authority switching has been received, with being superimposed on the endoscopic image G1.

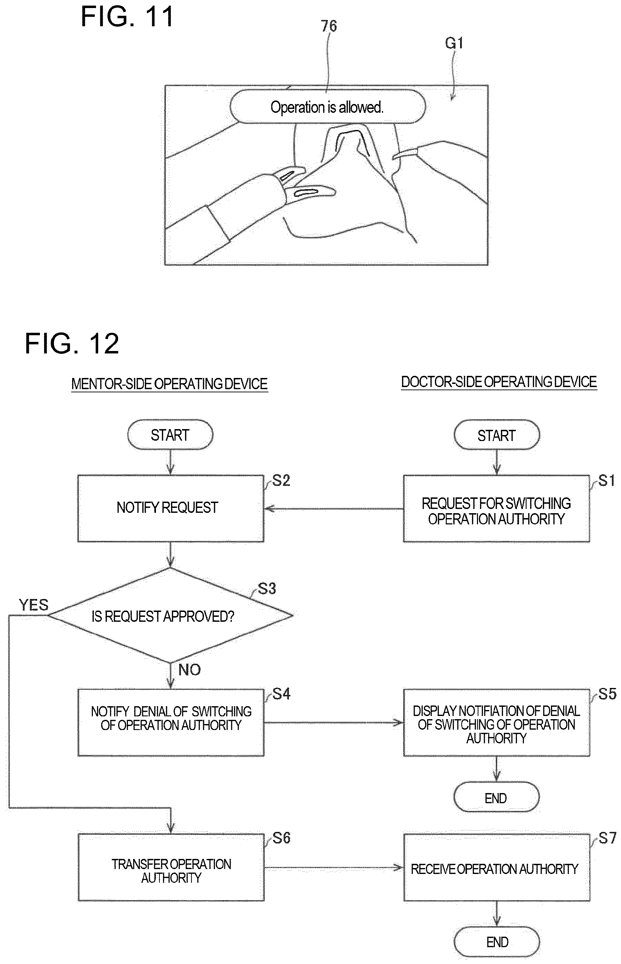

Further, as illustrated in FIG. 11, one of the doctor-side operating device 2 and the mentor-side operating device 3 that has the operation authority displays an indication 76 indicating that the one has the operation authority, so that the operator of the one can see that the one has the operation authority. For example, the doctor-side operating device 2 and the mentor-side operating device 3 display the indication 76 on the scope type display (24, 34) with being superimposed on the endoscopic image G1. Note that the process of superimposing the indication 76 on the endoscopic image G1 is performed by the controller 43.

With reference to a flowchart in FIG. 12, a control process for switching an operation authority will be described. Here, an example will be explained below in which the mentor-side operating device 3 has the operation authority, and then the operation authority is switched from the mentor-side operating device 3 to the doctor-side operating device 2. Each process of the flowchart is performed by the controller 28 of the doctor-side operating device 2 or the controller 38 of the mentor-side operating device 3.

As illustrated in FIG. 12, first, in step S1, by the touch panel 23 is operated, from the doctor-side operating device 2 to the mentor-side operating device 3, the switching request for the operation authority is transmitted. Then, in step S2, the mentor-side operating device 3 is notified of a notification that the switching request for the operation authority has been received. Then, in step S3, the mentor-side operating device 3 determines whether or not to approve the switching request for the operation authority. When the denial of the operation authority switching request is selected by operating the touch panel 33, the process proceeds to step S4. Then, in step S4, the mentor-side operating device 3 transmits to the doctor-side operating device 2 a notification of denial of switching the operation authority. Then, in step S5, the doctor-side operating device 2 displays an indication indicating that the notification of denial of switching the operation authority has been received. The control process is then terminated.

Further, in step S3, when approval of the switching request of the operation authority is selected by operating the touch panel 33, the process proceeds to step S6. In step S6, the operation authority is delegated from the mentor-side operating device 3 to the doctor-side operating device 2. In step S7, the operation authority is granted to the doctor-side operating device 2. The control process is then terminated.

### (Modifications)

Note that one or more embodiments disclosed herein should be considered as exemplary in all respects and do not limit the invention. The scope of the invention is indicated by claims, not by explanation of one or more embodiments described above, and includes equivalents to the claims and all alterations (modifications) within the same.

For example, in one or more embodiments described above, the case has been described in which each of the doctor-side operating device and the mentor-side operating device includes an annotation unit (the touch panel display and the voice communication device). However, the disclosure is not limited thereto. For example, only the mentor-side operating device may include an annotation unit (the touch panel display and the voice communication device).

Further, in one or more embodiments described above, the case has been described in which the control process of the disclosure are performed by the plurality of controllers. However, the disclosure is not limited thereto. For example, the control process of the disclosure may be performed by one controller.

For example, in one or more embodiments described above, the case has been described in which the scope type display is provided as a first display. However, the disclosure is not limited thereto. For example, a display other than a scope type display such as a flat panel display may be provided as a first display.

Further, in one or more embodiments described above, the case has been described in which the voice communication device is integrally provided with the scope type display as each of a first voice communication device and second voice communication device. However, the disclosure is not limited thereto. For example, as a first voice communication device or a second voice communication device, a voice communication device may be provided separately from a scope type display.

Further, in one or more embodiments described above, the case has been described in which the third voice communication device is integrally provided with the touch panel display. However, the disclosure is not limited thereto. For example, a voice communication device serving as a third voice communication device may be provided separately from the touch panel display. That is, as a third voice communication device, a voice communication device including a microphone and a speaker that are separated from the touch panel display may be provided. Further, in a case where a third voice communication device that is separated from the touch panel display is provided, each of the third voice communication device and the touch panel display may be connected to the mentor-side operating device via different interfaces.

Further, in one or more embodiments described above, the case has been described in which each of the doctor-side operating device and the mentor-side operating device includes the switching device. However, the disclosure is not limited thereto. For example, only one of the doctor operating device and the mentor operating device may include a switching device.

Further, in one or more embodiments described above, the case has been described in which the switching device is the touch panel disposed on the arm rest. However, the disclosure is not limited thereto. For example, the switching device may be a switch located on the armrest. Also, the switching device may be located at portion other than the armrest. For example, the switching device may be a switch provided on the first operating handle of the doctor-side operating device and/or the second operating handle of the mentor-side operating device. In this configuration, the doctor and/or the mentor can easily switch the operation authority with the switch at hand.

In one or more embodiments described above, a case has been described in which the controller 16 prohibits the acceptance of the instruction input through the touch panel display 51 when the mentor-side operating device 3 has the operation authority, whereas the controller 16 prohibits the acceptance of the instruction input through the touch panel display 61 when the doctor-side operating device 2 has the operation authority. However, the disclosure is not limited thereto. For example, the controller 16 may be configured such that the controller 16 permits acceptance of the instruction input through the touch panel display 51 when the mentor-side operating device 3 has the operation authority and the controller 16 permits acceptance of the instruction input through the touch panel display 61 when the doctor-side operating device 2 has the operation authority. In such a configuration, since the controller 16 permits the acceptance of the instruction input for generating the annotation image G2 through the touch panel display 51 and the touch panel display 61 regardless of whether the mentor-side operating device 3 and the doctor-side operating device 2 have the operation authority, it is possible to easily perform information sharing using the touch panel display 51 and the touch panel display 61.

Further, in one or more embodiments described above, a case has been described in which as a method of or a means for notifying that the switching request for switching the operation authority has been received, the indication 73 is displayed with being superimposed on the endoscopic image G1 on the scope type display (24, 34). However, the disclosure is not limited thereto. For example, as a means of notification, notification may be made using sound or voice via a voice communication device.

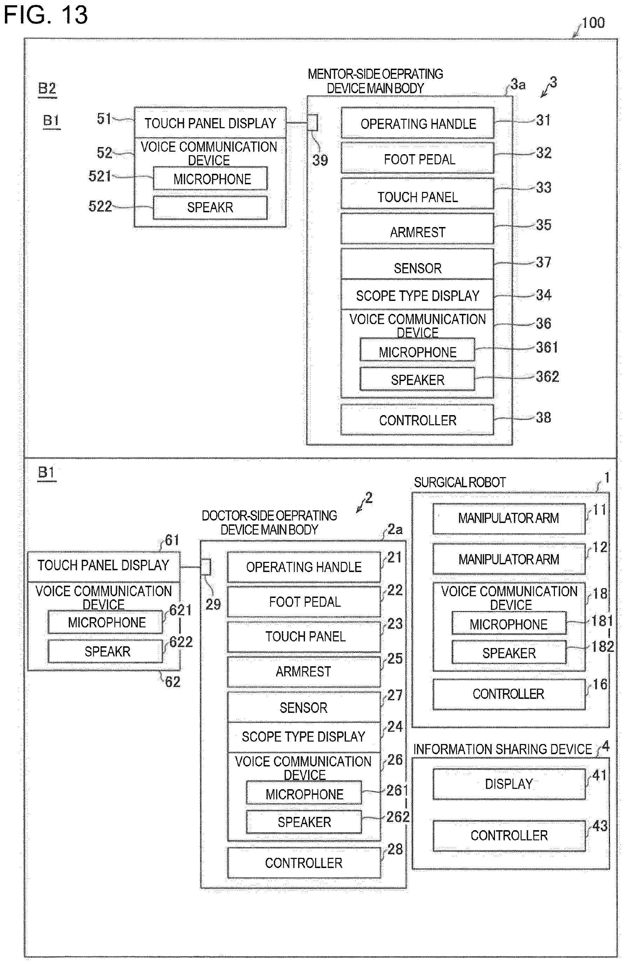

Further, in one or more embodiments described above, the case has been described in which the voice communication device is provided in the information sharing device. However, the disclosure is not limited thereto. For example, as an alternative to the voice communication device of the information sharing device, a voice communication device may be provided in the surgical robot. In a modification illustrated in FIG. 13, the surgical robot 1 includes a voice communication device 18 for voice communication with the voice communication device 26 of the doctor-side operating device 2. This also allows the assistant A3, who is assigned as a surgical staff near the surgical robot 1, to interact with the doctor A1. Further, in the modification, the voice communication device 18 is configured to perform voice communication with both the voice communication device 36 and the voice communication device 52. This also allows the mentor A2 and the clinical engineer A4 to hear the dialogue between the doctor A1 and the assistant A3 assigned as a surgical staff near the surgical robot 1. This also allows the assistant A3 to interact with the mentor A2 and the clinical engineer A4. The voice communication device 18 includes a microphone 181 and a speaker 182. The microphone 181 accepts voice input. The speaker 182 outputs sound. The voice communication device 18 is an example of a "fifth voice communication device."

Further, in one or more embodiments described above, the case has been described in which the mentor-side operating device 3 is provided in the second facility B2. However, the disclosure is not limited thereto. In the disclosure, a remote surgery support system may be configured such that a doctor-side operating device and a surgical robot are arranged in a second facility B2, the doctor-side operating device of the second facility B2 is configured, when a normal mode is selected from the mode selection, to operate the surgical robot of the second facility B2, and the doctor-side operating device of the second facility B2 is configured, when a remote mode is selected from the mode selection, to operate a surgical robot of the first facility B1 as a mentor-side operating device.

### (Aspects)

It may be appreciated by those skilled in the art that one or more embodiments described above may be specific examples of the following aspects.

(Item 1) A remote surgery support system including:
a first manipulator arm placed in a first facility and holding a first surgical instrument;
a second manipulator arm placed in the first facility and holding a second surgical instrument;
a third manipulator arm placed in the first facility and holding an endoscope;
a doctor-side operating device placed in the first facility and configured to be operated by a doctor to operate the first to third manipulator arms;
a mentor-side operating device placed in a second facility different from the first facility and configured to be operated by a mentor to operate the first to third manipulator arms via an external network; and
one or more controllers, wherein
the doctor-side operating device includes a first display configured to display an endoscopic image acquired by the endoscope, and a first voice communication device,
the mentor-side operating device includes:
   a second display configured to display the endoscopic image transmitted through the external network;
   a second voice communication device provided at a position that allows the mentor to perform voice communication with the first voice communication device while viewing the endoscopic image displayed on the second display;
   a touch panel display configured to display the endoscopic image transmitted through the external network and receive an instruction input for generating an annotation image; and
   a third voice communication device provided at a position closer to the touch panel display than the second display and configured to perform voice communication with the first voice communication device, and
the one or more controllers is configured to display on the first display the annotation image based on the instruction input received by the touch panel display with the annotation image being superimposed with the endoscopic image.

### (Item 2)

The remote surgery support system according to Item 1, wherein
at least one of the doctor-side operating device and the mentor-side operating device comprises a switching device configured to switch an operation authority for operating the first to third manipulator arms between the doctor-side operating device and the mentor-side operating device.

### (Item 3)

The remote surgery support system according to Item 1 or 2, wherein
the doctor-side operating device includes first operating handles configured to operate the first to third manipulator arms, and
the mentor-side operating device includes second operating handles configured to operate the first to third manipulator arms.

### (Item 4)

The remote surgery support system according to any one of Items 1 to 3, wherein
the second voice communication device and the third voice communication device are configured to perform voice communication with each other.

### (Item 5)

The remote surgery support system according to any one of Items 1 to 4, wherein
each of the first, second, and third voice communication devices includes a microphone and a speaker.

### (Item 6)

The remote surgery support system according to any one of Items 1 to 5, wherein
the third voice communication device is integrally provided with the touch panel display.

### (Item 7)

The remote surgery support system according to any one of Items 1 to 6, wherein
the mentor-side operating device includes an interface for connecting an external device, and
at least one of the third voice communication device and the touch panel display is connected as the external device to the mentor-side operating device via the interface.

### (Item 8)

The remote surgery support system of any one of Items 1 to 7, further comprising
a surgical robot that supports the first to third manipulator arms.

### (Item 9)

The remote surgery support system according to item 8, wherein
the surgical robot comprises an arm base supporting the first to third manipulator arms, a cart, an articulated robot that includes a distal end connected to the arm base and a proximal end supported to the cart.

### (Item 10)

The remote surgery support system according to Item 8 or 9, further comprising:
an information sharing device that is placed in the first facility and comprises a third display configured to display the endoscopic image acquired by the endoscope, and a fourth voice communication device configured to perform voice communication with the first voice communication device of the doctor-side operating device,
   wherein
the information sharing device is located closer to the surgical robot than to the doctor-side operating device.

### (Item 11)

The remote surgery support system according to Item 10, wherein
the fourth voice communication device is configured to perform voice communication with both of the second voice communication device and the third voice communication device.

### (Item 12)

The remote surgery support system according to any one of Items 8 to 11, wherein
the surgical robot includes a fifth voice communication device configured to perform voice communication with the first voice communication device of the doctor-side operating device.

### (Item 13)

The remote surgery support system according to Item 12, wherein
the fifth voice communication device is configured to perform voice communication with both of the second voice communication device and the third voice communication device.

### (Item 14)

The remote surgery support system according to any one of Items 1 to 13, wherein
the one or more controllers is located in the first facility and configured to display on the first display the annotation image based on the instruction input received via the external network, with the annotation image being superimposed with the endoscopic image.

### (Item 15)

The remote surgery support system according to Item 3 or any one of claims 4 to 14 dependent on claim 3, wherein
the one or more controllers is provided in the first facility and configured to display the annotation image based on the instruction input received via the external network , with the annotation image being superimposed with the endoscopic image on the first display.

The invention includes other embodiments or modifications in addition to one or more embodiments described above without departing from the spirit of the invention. The one or more embodiments described herein are to be considered in all respects as illustrative, and not restrictive. The scope of the invention is indicated by the appended claims rather than by the foregoing description. Hence, all configurations including the meaning and range within equivalent arrangements of the claims are intended to be embraced in the invention.

## Claims

1. A remote surgery support system comprising:
a first manipulator arm placed in a first facility and holding a first surgical instrument;
a second manipulator arm placed in the first facility and holding a second surgical instrument;
a third manipulator arm placed in the first facility and holding an endoscope;
a doctor-side operating device placed in the first facility and configured to be operated by a doctor to operate the first to third manipulator arms;
a mentor-side operating device placed in a second facility different from the first facility and configured to be operated by a mentor to operate the first to third manipulator arms via an external network; and
one or more controllers, wherein
the doctor-side operating device includes a first display configured to display an endoscopic image acquired by the endoscope, and a first voice communication device,
the mentor-side operating device includes:
a second display configured to display the endoscopic image transmitted through the external network;
a second voice communication device provided at a position that allows the mentor to perform voice communication with the first voice communication device while viewing the endoscopic image displayed on the second display;
a touch panel display configured to display the endoscopic image transmitted through the external network and receive an instruction input for generating an annotation image; and
a third voice communication device provided at a position closer to the touch panel display than the second display and configured to perform voice communication with the first voice communication device, and
the one or more controllers is configured to display on the first display the annotation image based on the instruction input received by the touch panel display with the annotation image being superimposed with the endoscopic image.

2. The remote surgery support system according to claim 1, wherein
at least one of the doctor-side operating device and the mentor-side operating device comprises a switching device configured to switch an operation authority for operating the first to third manipulator arms between the doctor-side operating device and the mentor-side operating device.

3. The remote surgery support system according to claim 1 or 2, wherein
the doctor-side operating device includes first operating handles configured to operate the first to third manipulator arms, and
the mentor-side operating device includes second operating handles configured to operate the first to third manipulator arms.

4. The remote surgery support system according to any one of claims 1 to 3, wherein
the second voice communication device and the third voice communication device are configured to perform voice communication with each other.

5. The remote surgery support system according to any one of claims 1 to 4, wherein
each of the first, second, and third voice communication devices includes a microphone and a speaker.

6. The remote surgery support system according to any one of claims 1 to 5, wherein
the third voice communication device is integrally provided with the touch panel display.

7. The remote surgery support system according to any one of claims 1 to 6, wherein
the mentor-side operating device includes an interface for connecting an external device, and
at least one of the third voice communication device and the touch panel display is connected as the external device to the mentor-side operating device via the interface.

8. The remote surgery support system according to any one of claims 1 to 7, further comprising
a surgical robot that supports the first to third manipulator arms.

9. The remote surgery support system according to claim 8, wherein
the surgical robot comprises an arm base supporting the first to third manipulator arms, a cart, an articulated robot that includes a distal end connected to the arm base and a proximal end supported to the cart.

10. The remote surgery support system according to claim 8 or 9, further comprising:
an information sharing device that is placed in the first facility and comprises a third display configured to display the endoscopic image acquired by the endoscope, and a fourth voice communication device configured to perform voice communication with the first voice communication device of the doctor-side operating device, wherein
the information sharing device is located closer to the surgical robot than to the doctor-side operating device.

11. The remote surgery support system according to claim 10, wherein
the fourth voice communication device is configured to perform voice communication with both of the second voice communication device and the third voice communication device.

12. The remote surgery support system according to any one of claims 8 to 11, wherein
the surgical robot includes a fifth voice communication device configured to perform voice communication with the first voice communication device of the doctor-side operating device.

13. The remote surgery support system according to claim 12, wherein
the fifth voice communication device is configured to perform voice communication with both of the second voice communication device and the third voice communication device.

14. The remote surgery support system according to any one of claims 1 to 13, wherein
the one or more controllers is located in the first facility and configured to display on the first display the annotation image based on the instruction input received via the external network, with the annotation image being superimposed with the endoscopic image.

15. The remote surgery support system according to claim 3 or any one of claims 4 to 14 dependent on claim 3, wherein
the second display is a scope type display,
the mentor-side operating device includes a sensor configured to detect whether or not the scope type display is in a state of use in which the scope type display is looked into, and
the one or more controllers is configured, when the sensor does not detect the state of use, to prohibit the second operating handle from operating the first to third manipulator arms and configured, when the sensor detects the state of use, to permit the second operating handle to operate the first to third manipulator arms.
